Europäisches Patentamt

European Patent Office

Office européen des brevets

(19)

(11) Numéro de publication: **0 242 272**
**A1**

(12) **DEMANDE DE BREVET EUROPEEN**

(21) Numéro de dépôt: 87400771.9

(22) Date de dépôt: 07.04.87

(51) Int. Cl.⁴: **C 12 N 5/00**
C 12 N 15/00, C 12 N 7/00

(30) Priorité: 08.04.86 FR 8604999

(43) Date de publication de la demande:
21.10.87 Bulletin 87/43

(84) Etats contractants désignés:
AT BE CH DE ES FR GB GR IT LI LU NL SE

(71) Demandeur: INSTITUT NATIONAL DE LA RECHERCHE
AGRONOMIQUE (INRA)
149, rue de Grenelle
F-75341 Paris Cedex 07 (FR)

CENTRE NATIONAL DE LA RECHERCHE SCIENTIFIQUE
(CNRS)
15, Quai Anatole France
F-75007 Paris (FR)

UNIVERSITE CLAUDE BERNARD - LYON 1
43, boulevard du 11 Novembre 1918
F-69622 Villeurbanne Cédex (FR)

(72) Inventeur: Jurdic, Pierre
6, rue Bonnand
F-69003 Lyon (FR)

Gandrillon, Olivier
59, rue de l'Abondance
F-69003 Lyon (FR)

Samarut, Jacques
169 bis, route de Genas
F-69100 Villeurbanne (FR)

Nigon, Victor Marc
9, rue Condorcet
F-69100 Villeurbanne (FR)

Benchaibi, Miloud
7, rue Jean-Claude Vivant
F-69100 Villeurbanne (FR)

(74) Mandataire: Warcoin, Jacques et al
Cabinet Régimbeau 26, avenue Kléber
F-75116 Paris (FR)

Le (Les) microorganisme(s) a (ont) été déposé(s) auprès CNCM sous le(s) numéro(s): Souche E. Coli 600RS P AEV-XJ12 no. I-494 ie 09.10.1985.

(54) **Procédé pour la préparation de cultures à long terme de cellules.**

(57) L'invention concerne un procédé pour préparer des cultures à long terme de cellules non transformées aviaires ou de mammifères, caractérisé en ce qu'on infecte une culture de cellules aviaires ou de mammifères avec un vecteur ou un système de vecteurs ne présentant pas de caractère oncogène pour lesdites cellules mais capable d'intégrer dans ces cellules un gène choisi parmi les gènes v-myb, v-ets ou v-erbA.

EP 0 242 272 A1

**Description**

## PROCEDE POUR LA PREPARATION DE CULTURES A LONG TERME DE CELLULES.

La présente invention concerne un nouveau procédé destiné à obtenir des cultures à long terme de cellules aviaires ou de mammifères non transformées.

Les cultures de cellules sont actuellement largement utilisées comme hôtes pour la préparation de vaccins ou de protéines par des techniques de génie génétique. Il existe, bien entendu, d'autres potentialités pour ce type de cellules qui ne sont encore aujourd'hui qu'à l'état de projet ou de recherche théorique.

Pour des raisons de commodité, il est bien entendu préférable de disposer de cultures à long terme, c'est-à-dire de cultures qu'il ne soit pas nécessaire de régénérer régulièrement et qui peuvent, dans ces conditions, donner pendant des durées très importantes des rendements importants.

Ces cultures, appelées le plus souvent "lignées immortelles", sont en général constituées par des cellules transformées, c'est-à-dire des cellules dont l'utilisation pour certaines applications ne peut être envisagée avec suffisamment de sécurité.

Il est, dans certains cas, possible de réaliser des lignées immortelles non transformées, mais leur élaboration est excessivement longue, délicate et incertaine.

C'est pourquoi la présente invention propose un nouveau procédé permettant de préparer de façon très efficace des cultures à long terme de cellules non transformées, aviaires ou de mammifères, caractérisé en ce qu'on infecte une culture de cellules aviaires ou de mammifères avec un vecteur ou un système de vecteur ne présentant pas de caractère oncogène pour lesdites cellules mais capable d'intégrer dans ces cellules un gène choisi parmi les gènes v-myb, v-ets et v-erbA.

Ces vecteurs ou systèmes de vecteur doivent être de types divers, il s'agira la plupart du temps de virus tels que les rétrovirus portant les gènes v-myb, v-ets et v-erbA avec leur virus assistant, ces virus pouvant être d'origine naturelle ou bien d'origine synthétique, c'est-à-dire avoir subi différents traitements par la méthode des ADN recombinants.

Parmi les systèmes de vecteur utilisables, il faut citer :
   1) le virus AMV
   2) le virus E26
   3) le virus XJ12

Les deux premiers virus sont des virus naturels qui sont déjà connus dans l'état de la technique, par contre le virus XJ12 est un virus dérivé du virus AEV dans lequel on a supprimé le gène v-erbB.

Ces différents virus peuvent être propagés au moyen de divers virus assistants tels que MAV-2, E26 AV, RAV-1 et RAV-2.

La construction du virus XJ12 a été décrite dans de précédents brevets : FR-A-84 15764 et EP-A-85401999.9.

Bien entendu, il est possible de prévoir d'autres types de vecteurs, notamment il est possible de prévoir des plasmides portant l'ADN de provirus susceptibles de s'intégrer comme les virus précédents et qui véhiculeront l'un des gènes v-myb, v-ets ou v-erbA.

En particulier, il est possible de prévoir l'utilisation de l'ADN d'un plasmide correspondant à un virus défectif, ainsi sans "plasmide assistant", il ne pourra pas y avoir production de virus

Les cultures selon l'invention pourront être sélectionnées, par exemple, grâce à leur capacité à croître sur milieu pauvre en sérum, par exemple milieu MEM, contenant moins de 1 % de sérum de veau foetal, par exemple 0,5 % de sérum de veau foetal.

De même, il est possible de les sélectionner grâce au fait que ces cellules développent des tapis cellulaires sous une couche d'agar mou pauvre en sérum.

Il est possible, grâce au procédé, d'obtenir des cultures de cellules de différents types, en particulier d'origine aviaire et notamment des fibroblastes, par exemple des fibroblastes d'embryons de poulets (CEF) .

L'invention concerne également les cultures à long terme de cellules ainsi obtenues qui pourront servir de cellules hôtes pour divers types de manipulations biologiques, notamment comme supports pour la culture de vaccins ou comme cellules hôtes pour l'expression de protéines codées par des vecteurs, quelle que soit leur origine, virale ou plasmidique par exemple.

Il est particulièrement intéressant, dans le cas où l'on désire faire exprimer une protéine par l'intermédiaire de ce type de cultures à long terme de cellules, de prévoir que les vecteurs ayant servi à intégrer les gènes mentionnés précédemment soient également capables d'intégrer,éventuellement sous forme multicopies, un gène codant pour une protéine industriellement intéressante et qui sera susceptible d'être exprimée par ladite cellule. On obtiendrait ainsi une culture à long terme de cellules capables de produire en quantité importante une protéine déterminée.

Les exemples suivants sont destinés à illustrer plus particulièrement d'autres caractéristiques et avantages de la présente invention.

Le milieu normal de croissance des CEF utilisé est un milieu MEM contenant 10 % de TPB ("tryptose phosphate broth"), des antibiotiques et de la fungizone plus des quantités variables de sérum de veau foetal.

## EXEMPLE 1

CROISSANCE DE FIBROBLASTES D'EMBRYONS DE POULETS INFECTES AVEC AMV ET E26 DANS UN MILIEU CEF NORMAL

Des fibroblastes d'embryons de poulets CEF secondaires, croissant dans un milieu normal contenant 6 % de sérum de veau foetal, ont été infectés avec différents virus.

Au 4ème passage, on ensemence $5.10^5$ cellules par plaque de 60 mm et ce moment est considéré comme jour 0 pour la courbe de croissance.

Les fibroblastes infectés avec AEV sont utilisés comme contrôle pour étudier la croissance des fibroblastes transformés.

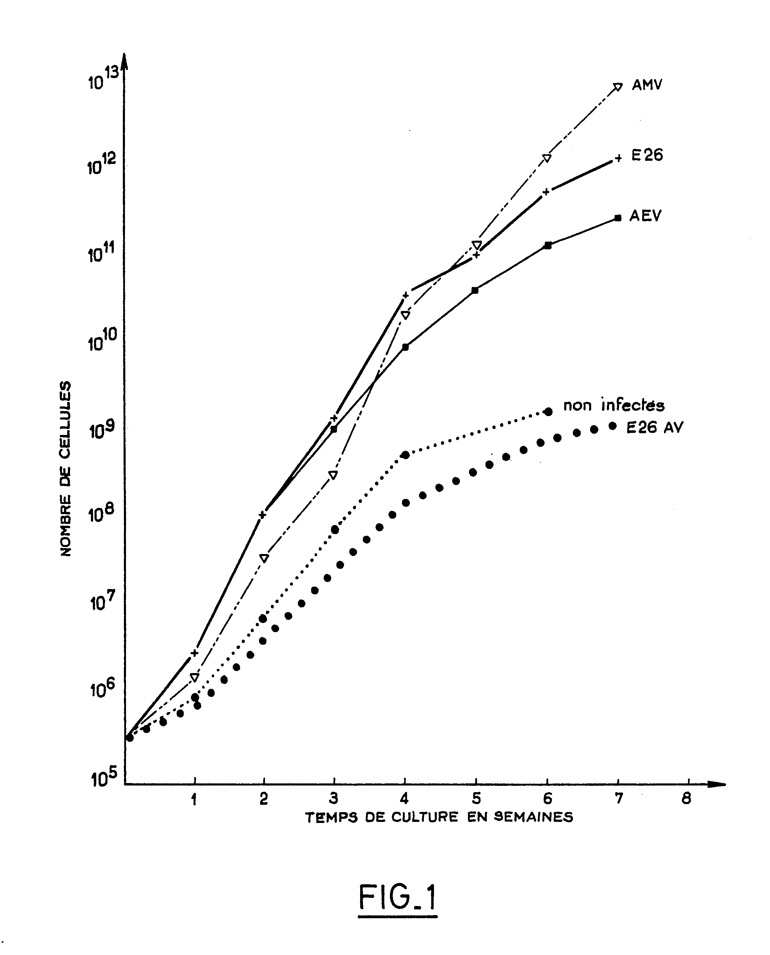

La figure 1, qui représente le nombre de cellules en fonction de la durée de la culture, permet de se rendre compte que les cellules infectées par E26 AV et que les cellules non infectées présentent un aspect de croissance typique, c'est-à-dire qu'après 4 ou 5 semaines, soit environ 12 passages, la croissance est réduite et les cellules sont en mauvaise santé et finalement meurent. Sur 5 semaines de croissance, le temps de doublement de la population est respectivement de 3,5 et 3,8 jours.

Par contraste avec ces résultats, les CEF infectés par AEV,E26 et AMV présentent un taux de croissance très supérieur. On peut remarquer :
- Que les CEF infectés par E26 et AMV présentent exactement le même type de croissance avec un temps de doublement de l'ordre de 2,1 et 2,3 jours, pendant environ 7 à 8 semaines. Ensuite, les cellules peuvent être maintenues en culture pendant une période d'au moins 6 mois sans mourir.
- Les fibroblastes infectés par AEV, avec un temps de doublement de 2,3 jours, ne croissent pas aussi vite que les CEF infectés par E26 et AMV.

On démontrera, ci-après, que les CEF infectés par E26 et AMV ne sont pas transformés, à la différence des fibroblastes infectés par AEV.

Il ressort de ces résultats que dans des conditions normales de croissance des CEF, celle-ci est clairement améliorée par infection avec AMV et E26 et non pas par les virus assitants correspondants.

Ces taux de croissance sont équivalents à ceux des fibroblastes qui ont été transformés. Il ne s'agit pas d'une stimulation limitée puisqu'elle peut durer de nombreuses semaines.

## EXEMPLE 2

### CROISSANCE DE FIBROBLASTES D'EMBRYONS DE POULETS INFECTES AVEC AMV ET E26 EN MILIEU PAUVRE

On utilise les mêmes cellules que dans l'exemple 1.

Au 4ème passage on ensemence $7,5.10^5$ cellules par plaque de 60 mm avec un milieu pour CEF contenant seulement 0,5 % de sérum de veau foetal au lieu des 6 % précédents.

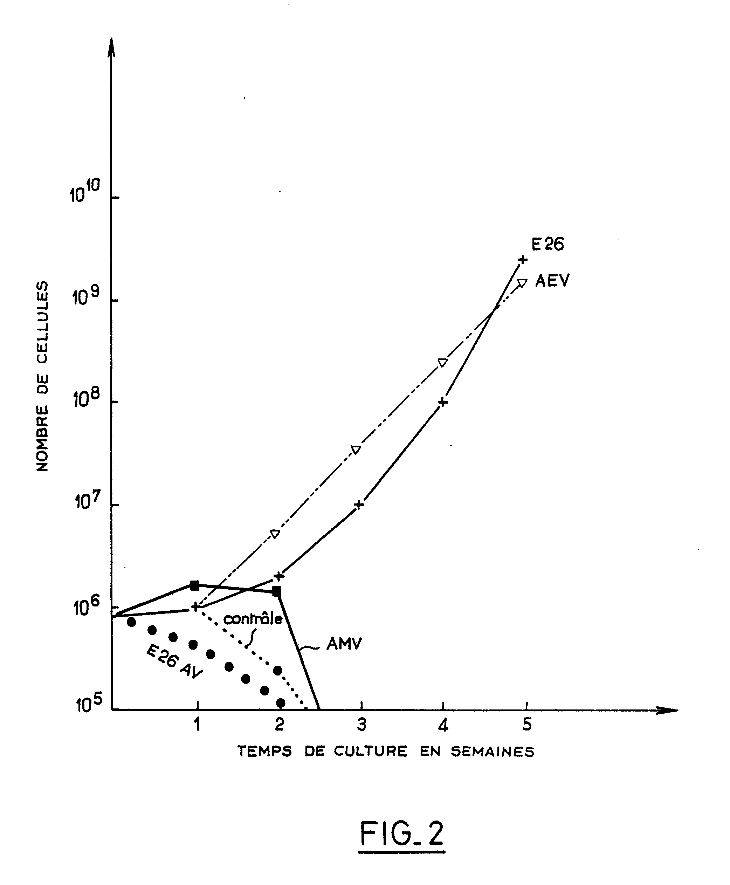

La croissance cellulaire correspondante est représentée à la figure 2.

Comme cela a été prévu, les CEF infectés par E26 AV, de même que les CEF non infectés, ne peuvent croître sur ce type de milieu et meurent après quelques jours de culture. La mort des cellules infectées par E26AV est plus rapide que la mort des cellules non infectées.

Par contre les CEF infectés par AMV peuvent être maintenus pendant 2 semaines sur ces cultures sans modification du nombre de cellules.

Les CEF infectés par E26 montrent une croissance soutenue en présence de seulement 0,5 % de sérum. Le temps de doublement de la population cellulaire est d'environ 3 jours, c'est-à-dire un peu plus long que dans le milieu normal.

## EXEMPLE 3

### FOYERS SOUS AGAR SEMI-SOLIDE PAUVRE EN SERUM

Dans ce test, une couche d'agar mou contenant du milieu à 0,5 % de sérum recouvre les cellules fraîchement ensemencées. Dans ces conditions, seuls les fibroblastes infectés par E26 et AEV développent des foyers de cellules normales.

Ce test rapide permet, dès la seconde semaine, après l'ensemencement, de distinguer les effets des virus mitogènes.

## EXEMPLE 4

### EFFETS MITOGENES DU GENE v-erbA SUR LES FIBROBLASTES D'EMBRYONS DE POULETS

Afin d'étudier les effets du gène v-erbA sur les fibroblastes d'embryons de poulets (CEF) on a infecté ces cellules avec le virus XJ12 qui a été construit à partir de l'AEV en remplaçant le gène v-erbB par le gène Neo. Afin de disposer d'un virus contrôle, on a créé le virus mutant XJ15 dans lequel le gène v-erbA a subi une délétion d'environ 500 nucléotides, le reste du génome viral étant identique à celui de XJ12. Ces deux virus sont multipliés par coinfection de CEF avec les virus helper RAV-1 ou RAV-2.

### Recherche de la protéine codée par v-erbA

Dans les virus AEV et XJ12 le gène v-erbA est fusionné à des séquences résiduelles du gène gag. L'ensemble code pour une protéine fusion de 75 kilodaltons appelée p75gag-erbA.

On a vérifié que des fibroblastes infectés par XJ12 produisent une protéine p75gag-erbA identique à celle produite par le virus AEV. Par contre, dans les CEF infectés par XJ15, cette protéine n'est pas détectée.

### Effet mitogène de XJ12 sur les CEF

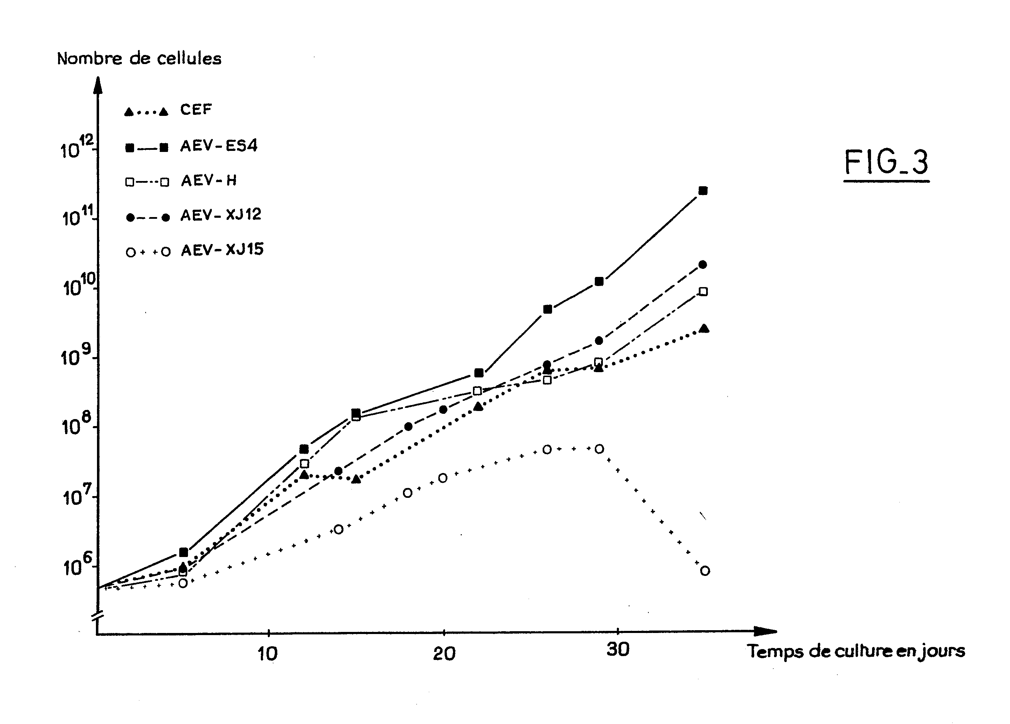

Des CEF ont été infectés par XJ12 (RAV-2) et XJ15 (RAV-2). La croissance des cellules a alors été analysée par dénombrement des CEF en milieu usuel (contenant 6 % de sérum de veau foetal, figure 3).

On constate que les fibroblastes infectés par XJ15 (RAV-2) montrent une croissance limitée à 3-4 semaines alors que ceux infectés par XJ12 (RAV-2) présentent une croissance beaucoup plus rapide et durable. Au bout de 5 semaines, les cultures infectées par XJ12 contiennent 10 000 fois plus de cellules que les cultures infectées par XJ15 et environ 10 fois plus que les CEF non infectés.

AEV-ES4 et AEV-H sont des virus AEV à activité transformante, AEV-ES4 contient v-erbA et v-erbB et AEV-H ne contient que v-erbB.

On peut donc mettre en évidence un effet mitogène du virus XJ12 dans des conditions où :
. le virus associé RAV-2 présente une cytotoxicité visible lors de l'infection par XJ15,
. les CEF non infectés sont cultivés dans des conditions de croissance optimales.

Croissance des CEF infectés en milieu pauvre en sérum

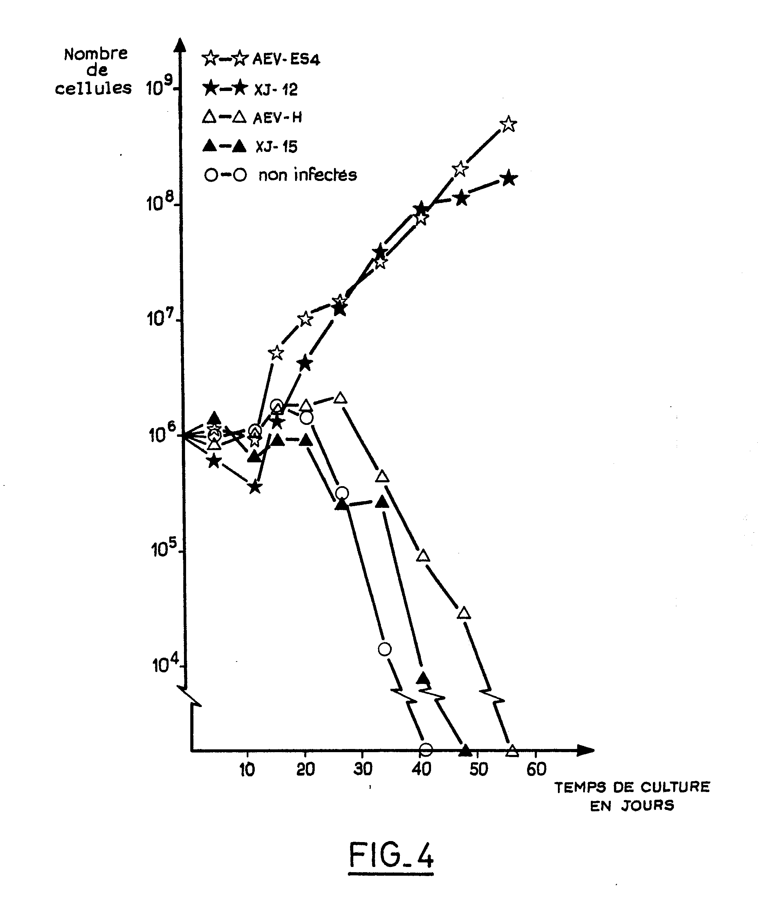

Des CEF sont infectés par les virus AEV-ES4 (contenant les séquences v-erbA et v-erbB), AEV-H (ne contenant que la séquence v-erbB), XJ12 et XJ15. Ces cellules sont ensuite cultivées en milieu pauvre en sérum (ne contenant que 0,5 % de sérum de veau foetal) (figure 4).

On constate que les cultures infectées par AEV-H et XJ15 ne se développent pas et s'éteignent au bout de 3-4 semaines. Par contre, les cultures infectées par AEV-ES4 et XJ12 présentent une croissance soutenue. Au bout de 9 semaines, le nombre de CEF infectés par XJ12 a été multiplié par 150.

Ces résultats montrent que l'expression constitutive de la protéine p75gag-erbA dans des CEF induit une prolifération soutenue de ces cellules dans un milieu pauvre en sérum.

Les CEF infectés par XJ12 ne sont pas transformés.

Les CEF infectés par AEV-ES4 présentent tous les caractères de cellules transformées : morphologie très fusiforme, contours très réfringents, croissance désordonnée en culture avec perte de l'inhibition de contact (aboutissant à des figures dites en "cross-cross" caractéristiques), développement de colonies en agar mou et taux d'incorporation d'hexose 10 fois supérieur à des CEF contrôles.

Les CEF infectés par XJ12 ne présentent, par contre, aucun caractère transformé : ces cellules ont une morphologie similaire à celle des CEF normaux, une croissance ordonnée en couche unicellulaire, ne forment pas de colonies dans l'agar et ont un taux d'incorporation d'hexose identique à celui de CEF contrôle.

Conclusion

Les CEF infectés par le virus XJ12 ne sont pas transformés mais diffèrent de CEF normaux par un potentiel multiplicatif accru ainsi que par la capacité à croître en milieu pauvre en sérum. On peut imaginer que l'expression de la protéine p75gag-erbA dans ces cellules leur impose un signal mitotique constitutif aboutissant à un maintien permanent de ces cellules en cycle. L'expression du gène v-erbA dans des cellules pourrait permettre la production de cultures non transformées à long terme.

EN RESUME

Les CEF infectés par AMV, E26 et XJ12 ne présentent pas les caractères de cellules transformées.

Un certain nombre de caractères sont généralement associés aux cellules qui subissent une transformation oncogène :
. croissance désordonnée en culture, perte de l'inhibition de contact,
. morphologie fusiforme ou arrondie, contours très réfringents,
. développement de colonies de cellules en suspension dans des milieux semi-solides,
. très forte incorporation d'hexose exogène,
. désorganisation totale du réseau de filaments d'actine dans les cellules,
. formation de tumeurs après injection in vivo.

Les CEF infectés par E26, AMV ou XJ12 présentent une morphologie plate voisine de celle des CEF normaux. Ils montrent une croissance ordonnée et sont sensibles à l'inhibition de contact en culture. Ils ne forment pas de colonies en milieu semi-solide et présentent un taux d'incorporation d'hexose faible voisin de celui des CEF normaux. Dans les CEF infectés par E26, les filaments d'actine n'ont pas complètement disparu mais sont plus discrets et nettement moins nombreux que dans les CEF normaux. Ce dernier caractère n'a pas encore été analysé dans les CEF infectés par XJ12. La formation de tumeurs après injection in vivo n'a pas encore pu être testée car ce test nécessite des souches de poulets particulières.

CONCLUSION

Les CEF infectés par E26, AMV ou XJ12 ne sont pas transformés mais présentent une vitesse de multiplication accrue ainsi qu'une augmentation de leurs potentiels de multiplication. En outre, les CEF infectés par E26 ou XJ12 acquièrent la capacité de proliférer en milieu pauvre en sérum. L'ensemble des caractères des CEF infectés par E26 ou XJ12 rapellent ceux généralement associés à la définition de cellules immortalisées chez les mammifères.

Le virus AEV XJ12 peut être obtenu par co-transformation de l'ADN du plasmide pAEV-XJ12 et de l'ADN contenant les génomes de virus helper RAV-1 ou RAV-2 sur fibroblastes de poulets, on récupère alors le virus AEV-XJ12(RAV-2).

EXEMPLE 5

TRANSFERT PAR TRANSFECTION DU GENE RETROVIRAL v-erbA, ANIME PAR DES PROMOTEURS RETROVIRAUX, DANS DES FIBROBLASTES d'EMBRYON DE POULET

Des fibroblastes secondaires d'embryon de poulet ont été transfectés d'une part avec le plasmide pXJ12 portant le gène v-erbA et le gène Neo, d'autre part avec le plasmide pTXN portant uniquement le gène Neo. Les cultures sont ensuite mises en présence de G418 pendant 5 jours pour sélectionner les cellules ayant intégré ces génomes partiellement viraux sous une forme fonctionnelle. Les génomes transfectés étant défectifs et l'opération ne comportant pas de helper, les cellules ainsi obtenues ne produisent pas de virus et paraissent, de ce point de vue, envisageables pour une utilisation pratique.

Sur $5 \times 10^5$ fibroblastes transfectés avec 7,5pg d'ADN pXJ12, 72 clones résistants au G418 ont été isolés et poolés. Sur un même nombre de fibroblastes transformés avec 7,5 pg d'ADN pTXN, 30 clones résistants au G418 ont été isolés et mélangés. Les cultures pXJ12 et pTXN ont été sous cultivées régulièrement (1 à 2 passages par semaine).

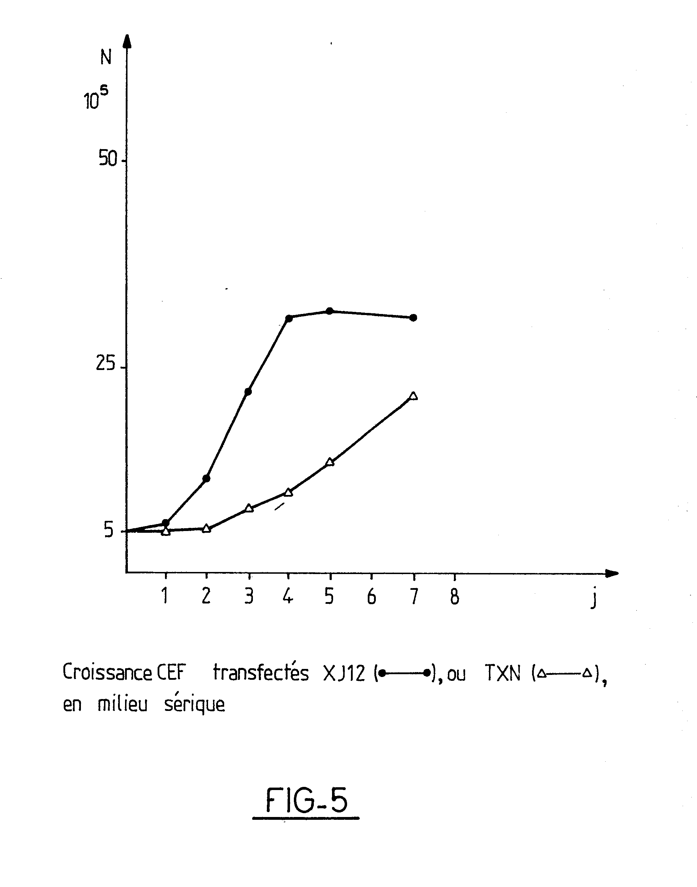

Une analyse cinétique de la croissance de ces cellules a été réalisée après environ 30 générations cellulaires pour pXJ12 et 25 générations cellulaires pour pTXN. Les courbes de croissance présentées sur la figure 5 montrent que les fibroblastes pXJ12 présentent une croissance plus rapide que fibroblastes pTXN. En particulier, les fibroblastes pXJ12 présentent une latence très courte après l'ensemencement. Ces résultats sont identiques à ceux obtenus précédemment avec les fibroblastes infectés par le virus XJ12.

Le comportement des fibroblastes pTXN est caractéristique de fibroblastes normaux qui arrivent en sénescence. Ces cellules n'ont pu être maintenues par la suite. Les fibroblastes pXJ12 sont conservés en ampoules maintenues en azote liquide. Ces cellules redémarrent très bien en culture après décongélation, et ont été sous cultivées ensuite pendant 3-4 passages. On ne sait pas combien de passages au total ces cellules pourraient encore supporter.

De toute évidence, cette expérience confirme que le génome XJ12 et dans celui-ci, vraisemblablement, le gène v-erbA, augmentent la survie des fibroblastes de poulet. On ne possède pas encore la preuve que ces cellules soient immortalisées.

La souche suivante a été déposée à la Collection Nationale de Cultures de Microorganismes de l'Institut Pasteur, 28 rue du Docteur-Roux, 75724 PARIS CEDEX 15 :
- Souche E. coli 6OORS pAEV-XJ12 no I-494 le 9 octobre 1985.

**Revendications**

1) Procédé pour préparer des cultures à long terme de cellules non transformées aviaires ou de mammifères, caractérisé en ce qu'on infecte une culture de cellules aviaires ou de mammifères avec un vecteur ou un système de vecteurs ne présentant pas de caractère oncogène pour lesdites cellules mais capable d'intégrer dans ces cellules un gène choisi parmi les gènes v-myb, v-ets ou v-erbA.

2) Procédé selon la revendication 1, caractérisé en ce que les systèmes de vecteurs sont choisis parmi les virus portant les gènes v-myb, v-ets et v-erbA et leurs virus assistants.

3) Procédé selon la revendication 2, caractérisé en ce que les systèmes de vecteurs sont choisis parmi :
- les virus AMV et son virus assistant MAV2,
- le virus E26 et son virus assistant E26AV,
- le virus XJ12 et son virus assistant RAV-1 ou RAV-2.

4) Procédé selon l'une des revendications 1 à 3, caractérisé en ce que le vecteur est un plasmide portant l'un des gènes v-myb, v-ets ou v-erbA.

5) Procédé selon la revendication 4, caractérisé en ce que le plasmide est défectif et en ce qu'on n'utilise aucun plasmide helper.

6) Procédé selon la revendication 5, caractérisé en ce que le plasmide est le plasmide pXJ12.

7) Procédé selon l'une des revendications 1 à 6, caractérisé en ce que le vecteur ou le système de vecteurs comporte, en outre, un autre gène capable de s'exprimer dans lesdites cellules.

8) Procédé selon la revendication 7, caractérisé en ce que l'autre gène code pour une protéine d'intérêt industriel.

9) Procédé selon l'une des revendications 1 à 8, caractérisé en ce que les cellules sont des fibroblastes aviaires.

10) Procédé selon la revendication 9, caractérisé en ce que les cellules sont des fibroblastes d'embryons de poulets.

11) Procédé selon l'une des revendications 1 à 10, caractérisé en ce que la sélection des cultures à long terme de cellules est effectuée sur un milieu pauvre en sérum.

12) Procédé selon la revendication 11 caractérisé en ce que le milieu pauvre en sérum contient moins de 1 % de sérum.

13) Culture à long terme de cellules non transformées aviaires ou de mammifères obtenue par la mise en oeuvre du procédé selon l'une des revendications 1 à 12.

FIG_1

FIG. 2

Nombre de cellules

▲····▲ CEF
■——■ AEV–ES4
□—··□ AEV–H
●——● AEV–XJ12
○+·○ AEV–XJ15

FIG_3

$10^{12}$

$10^{11}$

$10^{10}$

$10^9$

$10^8$

$10^7$

$10^6$

10   20   30   Temps de culture en jours

FIG_4

Croissance CEF transfectés XJ12 (●———●), ou TXN (△———△), en milieu sérique

## FIG-5

## Office européen des brevets

# RAPPORT DE RECHERCHE EUROPEENNE

Numéro de la demande

EP 87 40 0771

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | VIROLOGY, vol. 130, no. 1, 1983, pages 155-178, Academic Press, Inc.; L. SEALY et al.: "Site-specific mutagenesis of avian erythroblastosis virus: erb-B is required for oncogenicity" * Page 164, colonne de gauche * | 1,2,5, 7,8,13 | C 12 N 5/00<br>C 12 N 15/00<br>C 12 N 7/00 |
| | --- | | |
| A | CELL, vol. 32, janvier 1983, pages 227-238, MIT; L. FRYKBERG et al.: "Trasforming capacities of avian erythroblastosis virus mutants deleted in the erbA or erbB oncogenes" * Page 232 * | 1,2,5, 7,8,13 | |
| | --- | | |
| A | DNA, vol. 4, no. 1, janvier 1985, pages 23-31, Mary Ann Liebert, Inc.; J.J. KOPCHICK et al.: "Use of avian retroviral-bovine growth hormone DNA recombinants to direct expression of biologically active growth hormone by cultured fibroblasts" * Résumé * | 1,2,5, 6 | **DOMAINES TECHNIQUES RECHERCHES (Int. Cl.4)**<br><br>C 12 N<br>C 12 P |
| | ----- | | |

Le présent rapport de recherche a été établi pour toutes les revendications

| Lieu de la recherche | Date d'achèvement de la recherche | Examinateur |
|---|---|---|
| LA HAYE | 03-07-1987 | CUPIDO M. |